# EUROPÄISCHE PATENTSCHRIFT

(11) **EP 0 858 831 B1**
(45) Veröffentlichungstag und Bekanntmachung des Hinweises auf die Patenterteilung: **04.04.2001**
(21) Anmeldenummer: 97121567.8
(22) Anmeldetag: 08.12.1997
(51) Int. Cl.: B01J 20/32, C07K 14/765, C07K 17/02, C07K 17/06

(54) **Vorrichtung zur Reinigung proteinhaltiger Lösungen, Verfahren zur Herstellung eines Trägermaterials für die Vorrichtung und Verwendung der Vorrichtung**
Apparatus for the purification of proteic solutions, process to prepare a support material for the above apparatus and its use
Appareillage pour la purification de solutions protéiques, procédé pour la préparation d'un matériau de support pour le susdit appareillage et son utilisation

(30) Priorität: 12.02.1997 DE 19705366
(43) Veröffentlichungstag der Anmeldung: 19.08.1998
(73) Patentinhaber: Fresenius AG, 61350 Bad Homburg (DE)
(72) Erfinder: Otto, Veit Dr., 66606 St. Wendel (US); Schulze, Stefan, 61348 Bad Homburg (DE); Zimmermann, Michael, 66606 St. Wendel (DE)
(74) Vertreter: Laufhütte, Dieter, Dr.-Ing.

(56) Entgegenhaltungen:
- EP-A- 0 028 937
- WO-A-89/10377
- US-A- 4 323 486
- CHEMICAL ABSTRACTS, vol. 106, no. 8, 23.Februar 1987 Columbus, Ohio, US; abstract no. 55851, XP002067502 & R SIPEHIA ET AL.: "Enhanced albumin binding to polypropylene beads via anhydrous ammonia gaseous plasma" BIOMATERIALS, Bd. 7, Nr. 6, 1986, Seiten 471-473,

## Beschreibung

Die Erfindung betrifft eine Vorrichtung zur Reinigung proteinhaltiger Lösungen, wie Blut, Blutplasma oder Zellkulturmedien, ein Verfahren zur Herstellung eines Trägermaterials für die vorgenannte Vorrichtung und die Verwendung dieser Vorrichtung.

Es ist bereits bekannt, daß eine Reihe von Stoffwechseltoxinen, wie zum Beispiel Merkaptane, freie Fettsäuren, unkonjugiertes Bilirubin sowie die Endotoxine gramnegativer Bakterien und viele exogene Toxine, insbesondere Medikamente, wie zum Beispiel Nortriptylin, Amitryptilin, Diazepam, Bromazepam etc. im Blutkreislauf nahezu vollständig an Proteine gebunden sind. Diese Bindung erfolgt bevorzugt an die Albuminfraktion des Blutplasmas. Aufgrund der Molekülgröße und der starken Bindungswechselwirkung des Protein-Toxinkomplexes lassen sich albumingebundene Toxine mit herkömmlichen Blutreinigungsverfahren, wie beispielsweise der Hämodialyse, nicht oder nur in sehr geringem Umfang selektiv aus dem Blut entfernen.

Es sind allerdings schon Verfahren zur Blutreinigung bekannt geworden, mit denen sich proteingebundene Toxine aus dem Blut oder dem Plasma entfernen lassen. Ein bekanntes Verfahren stellt die Hämoperfusion mit Aktivkohle oder lonentauscherharzen dar. Diese Adsorbermaterialien zeigen für viele proteingebundene Toxine messbare Eliminationsraten (Rosenbaum, J.L. et al. 1980: Current status of hemoperfusion in toxicology. Clin Toxicol 17: 493). Nachteilig sind bei den genannten Adsorbentien die weitgehend unspezifischen Bindungseigenschaften. So werden neben den albumingebundenen Toxinen unerwünschterweise Hormone, Wachstumsfaktoren und Zellen wie beispielsweise Thrombozyten mitentfernt, was zu Nebenwirkungen und Komplikationen führen kann.

Die WO 94/21363 beschreibt ein Dialyseverfahren, mit welchem sich albumingebundene Substanzen aus dem Blut entfernen lassen. Hierbei wird eine asymmetrische Dialysehohlfaser im wesentlichen auf der blutabgewandten Seite (in der porösen Stützschicht) mit Albumin beschichtet. Des weiteren werden die Hohlfasern außenseitig mit einer Albuminlösung umspült, wobei diese Albuminlösung in einem Kreislauf zusätzlich über eine Aktivkohle- bzw. lonenaustauscherpatrone und durch einen weiteren Dialysator geleitet wird, um die transportierten Toxine aus dem Dialysat zu entfernen. Die mit Toxinen beladenen Albuminmoleküle des Blutes sind in dieser Anordnung durch eine innere, albuminundurchlässige Trennschicht der Dialysefaser von den toxinfreien Albuminmolekülen der Stützschicht räumlich getrennt. Die albuminundurchlässige Trennschicht ist für die Funktion dieser Anordnung unerläßlich, um eine Rückdiffusion der im wesentlichen frei beweglichen Toxin-Albuminkomplexe zu verhindern und so einen gerichteten Transport von Toxinen aus dem Blut in das Dialysat aufrechtzuerhalten.

Um aus dem Blut entfernt zu werden, müssen die Toxine die albuminundurchlässige Trennschicht passieren. Dies ist besonders für Entfernung großer Toxinkomplexe, wie zum Beispiel Endotoxinkomplexe gramnegativer Bakterien, nachteilig. Diese lassen sich mit dem beschriebenen Verfahren nicht aus dem Blut entfernen. Des weiteren zeigt diese Anordnung ebenfalls keine selektive Entfernung von Stoffen, da sämtliche Substanzen, die aufgrund ihrer niedrigen Molekülgröße die innere Trennschicht der Dialysefaser passieren können mitentfernt werden.

Die WO 95/04559 beschreibt eine Anordnung mit einem Membranfilter zur Elimination von Toxinen aus proteinhaltigen Flüssigkeiten, insbesondere Blut. Die zu reinigende Flüssigkeit, z. B. Blut, wird durch das Lumen eines hohlfaserförmigen Membranfilters geleitet, dessen Außenseite von einer Reinigungssuspension umflossen wird, die feste, Toxine bindende Partikel enthält. Die Reinigungssuspension wird über eine Zentrifugalpumpe in Bewegung gehalten und bewirkt lokal unterschiedliche transmembrane Druckdifferenzen an dem Membranfilter, wodurch ein lokaler Flüssigkeitsaustausch durch die Filterwandung stattfindet. Mit der Flüssigkeit transportierte Toxine kommen so in Kontakt mit den adsorbierenden Partikeln, während gleichzeitig gereinigte Flüssigkeit in den Blutstrom zurückfließt. Auch diese Anordnung hat den Nachteil, daß die Toxinkomplexe die Filterwandung passieren müssen, um aus der zu reinigenden Flüssigkeit entfernt zu werden.

Aus.der J. of Clinical Investigation, Vol. 53 (März 1974), Seiten 778-785 ist bereits ein mit Albumin beschichteter Adsorber bekannt, der zur Entfernung proteingebundener Metaboliten oder Toxine aus dem Blut benutzt wurde. Der bekannte Adsorber ist jedoch weder hämokompatibel, noch heißdampfsterilisierbar, wodurch keine therapeutische Nutzung möglich ist. Die Kopplung des Albumins erfolgt über Cyanbromid, welches als Verunreinigung dem Endprodukt anhaften kann, und, selbst in geringen Konzentrationen, ein starkes Gift darstellt. Auch ist das Trägermaterial nicht geeignet, eine Komplementaktivierung oder Thromboszytenadhäsion zu verhindern.

Aufgabe der Erfindung ist es, eine Vorrichtung zu schaffen, mit der proteingebundene Toxine unabhängig von ihrer Größe aus einer proteinhaltigen Lösung, wie beispielsweise Blut, Blutplasma oder Zellkulturmedien, selektiv und effektiv entfernt werden.

Diese Aufgabe wird durch eine Vorrichtung nach Anspruch 1 gelöst, die ein biokompatibles Trägermaterial aus Kunststoffmaterialien aufweist, wobei das Kunststoffmaterial aus folgender Gruppe ausgewählt ist: Polyacrylate, Polymethacrylate, Polysulfone, Polyethersulfone, welche Amine bzw. Amide tragen und die mittels Peptidbindung mit Albumin kovalent beschichtet sind. Dieses erfindungsgemäß in der Vorrichtung eingesetzte Trägermaterial weist besonders gute Eigenschaften als Adsorbens auf.

Besonders vorteilhaft ist es, wenn das Albumin über eine Mehrpunktbindung an das Kunststoffmaterial gebunden ist. Hierdurch wird die Sterilisierbarkeit des Absorbers sichergestellt.

Das Trägermaterial kann in Form von Schüttungen von Beads aber auch in Form von Membranen in Flach- oder Hohlfaserform bzw. in Form einer Folie eingesetzt werden. Soweit Beads eingesetzt werden, können diese vorteilhaft einen Durchmesser zwischen 10 bis 500 µm haben und in einer Säule mit mindestens einem Zu- und einem Ablauf in Form einer perfundierbaren Schüttung eingefüllt sein.

Gemäß einem vorteilhaften Verfahren zur Herstellung des in der erfindungsgemäßen Vorrichtung einzusetzenden Trägermaterials wird eine Mehrpunktbindung über ein Kopplerreagenz erzeugt, das lediglich die Carbonylfunktion des Albumins aktiviert und anschließend wieder abgespalten wird. Als Kopplerreagenz kann Carbodiimid oder N-Hydroxysuccinimid im Überschuß eingesetzt werden. Das Kopplerreagenz wirkt wie ein "Katalysator", da es lediglich die Carbonylfunktion des Albumins aktiviert. Nach der Reaktion wird das Kopplerreagenz wieder abgespalten, beispielsweise im Fall von Carbodiimid als ein Dialkylharnstoff.

Vorteilhaft liegt das molare Verhältnis der reaktiven Gruppen des Trägers der Albuminkonzentration von 50 bis 500 zu 1. Das Kopplerreagenz wird vorteilhaft bezogen auf die reaktiven Gruppen des Trägermaterials in gleicher bis zehnfacher molarer Konzentration eingesetzt. Dadurch wird eine Mehrpunktanbindung erreicht, die die erwünschte Dampfsterilisierbarkeit erlaubt.

Erfindungsgemäß kann die vorgenannte Vorrichtung zur Entfernung von Toxinen aus menschlichem Vollblut, aber auch aus menschlichem Plasma oder Zellkulturmedien verwendet werden.

### Erfindungsgemäßes Beispiel 1:

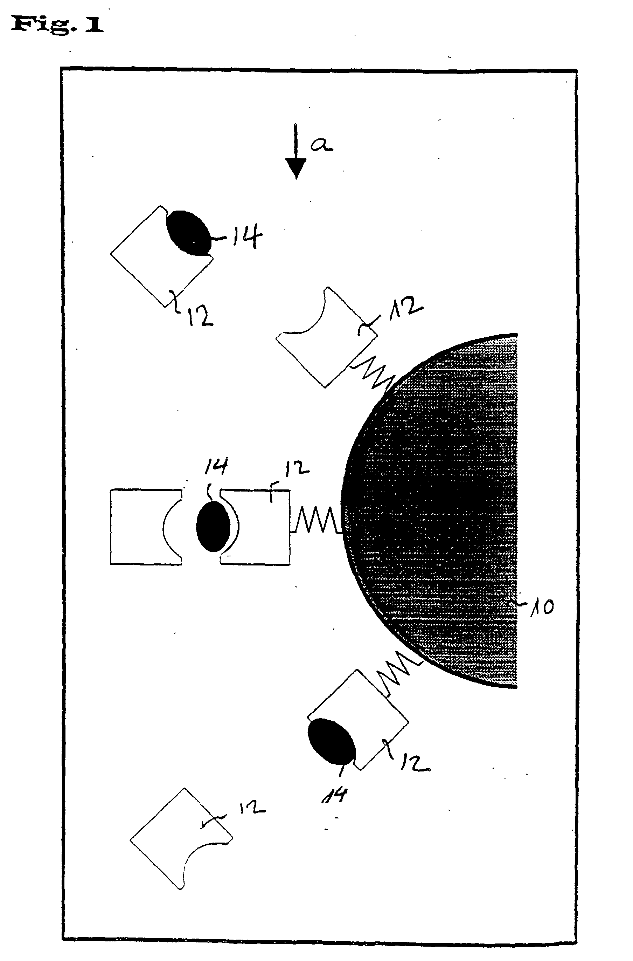

Kunststoffbeads, beispielsweise aus Polymethacrylat oder Polyhydroxymethacrylat (z.B. EUPERGIT^{R}, Fa. Röhm), mit einem Durchmesser von 10 bis 500 µm werden aminiert und mit gereinigtem Humanalbumin mittels geeigneter Kopplungsreagentien kovalent beschichtet und in eine Säule gefüllt. Die so hergestellte Säule wird in einem extrakorporalen Kreislauf mit Blut perfundiert. In Fig. 1 ist der Mechanismus schematisch dargestellt. Auf einem Kunststoffbead 10 ist Humanalbumin 12 kovalent gebunden. Blut perfundiert in Pfeilrichtung a durch die Säule, so daß toxin- bzw. albumingebundenes Toxin 14 an der beschichteten Kunststoffbeadoberfläche vorbeiströmt. Im Zuge der Perfusion wird das immobilisierte Albumin 12 Toxine 14 mit Albuminaffinität aus dem Blut adsorbieren.

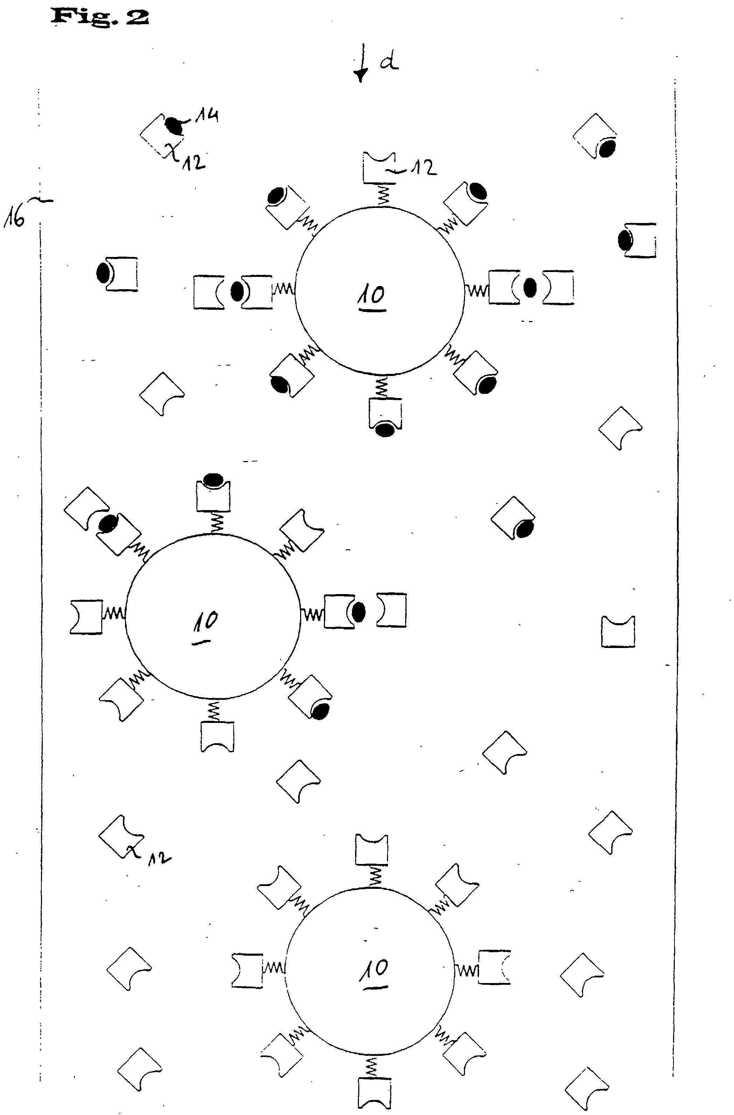

In Fig. 2 ist eine Säule 16 angedeutet, die in Pfeilrichtung d mit proteinhaltiger Lösung durchströmt wird. Diese enthält an Albuminmoleküle 12 gebundene Toxine 14. Die Toxine 14 werden im Verlauf der Strömung an das immobilisierte Albumin 12 auf den Oberflächen der Kunststoffbeads 10 abgegeben. Im unteren Bereich der Fig. 2 weisen die Albuminmoleküle 12 keine Toxine mehr auf, da diese quantitativ abgegeben wurden. Der in der Fig. 2 dargestellte Mechanismus entspricht demjenigen, der in Beispiel 1 näher erläutert ist.

### 1. Reaktion der kovalenten Albuminbeschichtung

20 g Oxiran-Acrylharzbeads (z.B. EUPERGIT^{R}, Fa. Röhm) mit einem mittleren Durchmesser von ca. 200 µm und mit einem mittleren Oxirangehalt von 0,2 mmol/g werden mit 240 ml 13%iger Ammoniaklösung 6 h bei Raumtemperatur aminiert. Nach Abschluß der Inkubation und vollständiger Entfernung der Ammoniaklösung durch mehrmaliges Waschen mit destilliertem Wasser (Kontrolle des pH-Wertes), werden die Beads in 120 ml 20 mM Natriumphosphat-Puffer pH 4,8 und 1 % (w/v) Serumalbumin (Bovine Serum Albumin fettsäurefrei, Sigma) resuspendiert. Der Ansatz wird in Eiswasser auf 4 bis 6°C abgekühlt. Die Kopplung erfolgt durch langsame Zugabe von 60 ml 5%iger (w/v) EDC-Lösung in Natriumphosphat-Puffer pH 4,8 (EDC = 1-Ethyl-3-(3-dimethylaminopropyl)-carbodiimid). Nach Zugabe des Carbodiimides wird der Ansatz 4 h in Eiswasser geschüttelt. Anschließend wird der Kopplungsreagenz- und Proteinüberschuß durch mehrmaliges Waschen mit eiskalter 0,9%iger Natriumchlorid-Lösung entfernt. Die so vorbehandelten Beads werden abschließend in 20 mM Natriumphosphat-Puffer pH 7,4 resuspendiert und die Proteinkopplungsausbeute bestimmt.

Typische Kopplungsausbeuten liegen mit BSA bei 8 bis 10 mg Protein/g Beads.

### 1.1. Adsorption von Fettsäuren aus Humanplasma im Batch-Verfahren

Aus frisch entnommenem ACD (ACD = Natriumcitrat/Citrat-Glucoselösung) anticoaguliertem Vollblut gesunder Spender wird durch Zentrifugation mit 1500 x g für 15 Minuten Plasma gewonnen. Der Fettsäuregehalt des Plasmas wird bestimmt.

In Eppendorfgefäßen werden nach Abschnitt 1 hergestellte Albumin-Acrylharzbeads sedimentiert und der Überstand an Natriumphosphat-Puffer entfernt. Genau 0,1 ml Sediment wird anschließend mit 0,5 ml Plasma versetzt. Der Ansatz wird 1h bei 37°C auf einem Schüttler inkubiert. Nach Abschluß der Inkubation läßt man die Albumin-Acrylharzbeads erneut sedimentieren und entnimmt aus dem Plasmaüberstand eine Probe zur Fettsäurebestimmung. Der restliche Überstand wird entfernt und die Beads werden dann 6 mal mit je 1 ml 20 mM Natriumphosphat-Puffer pH 7,4 gewaschen. Gemessen wird der Fettsäuregehalt des Ausgangsplasmas, des Plasmaüberstandes nach erfolgter Adsorption sowie die direkt an die Beads gebundene Fettsäure. Die Fettsäurebestimmung (Shimizu, S. et al. 1980: Anal. Biochem.107: 193-198) erfolgt photometrisch mittels eines käuflichen enzymatischen Farbtestes (Boehringer, Mannheim). Zur Ermittlung der albuminspezifischen Bindung wurden in allen Versuchen Kontrollbeads ohne Albuminbeschichtung unter analogen Bedingungen mitlaufen gelassen.

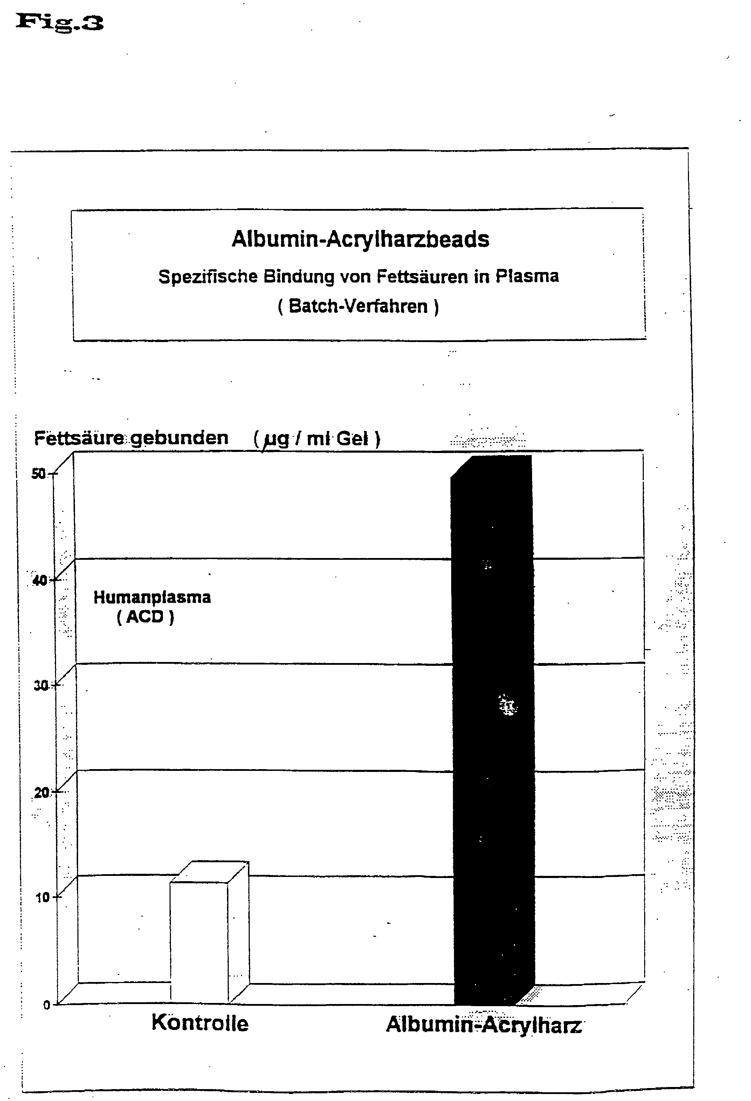

Tabelle 1 und Fig. 3 fassen die typischerweise ermittelten Ergebnisse des beschriebenen Versuches zusammen. Mit Albumin beschichtete Acrylharz-Beads zeigen gegenüber der Kontrolle eine deutlich erhöhte Bindung von Fettsäuren.

**Tabelle 1**

| Adsorptive Entfernung von Fettsäuren aus Humanplasma mit Albumin-Acrylharzbeads im Batch-Verfahren. | | | |
|---|---|---|---|
| **Probenart n=10** | **Extinktion (546nm)** | **Fettsäurekonz. (mmol/l)** | **Fettsäure gebunden (ug/ml Beads)** |
| Ausgangsplasma | 0,927 | 0,88 | - |
| Plasmaüberstand Albuminbeads | 0,630 | 0,60 | - |
| Plasmaüberstand Kontrolle | 0,803 | 0,76 | - |
| Albumin-Beads | 0,430 | 0,25 | 49,6 |
| Kontrollbeads | 0,098 | 0,05 | 11,3 |

### 1.2. Adsorption von Endotoxinen aus Humanplasma mittels albuminbeschichteter Acrylharz-Beads.

Genau 5 ml Albumin-Acrylharzbeads, hergestellt wie in Absatz 1 beschrieben, werden in eine Säule gefüllt. Die möglichst luftblasenfrei gepackte Säule wird zunächst mit 50 ml 0,5%iger (w/v) pyrogenfreier Desoxycholat-Lösung (Natrium-Desoxycholat, Fluka) mit einer Flußgeschwindigkeit von 5 ml/min perfundiert. Mit diesem Schritt werden eventuell an den Oberflächen haftende Endotoxine entfernt. Desoxycholatreste werden mit 1000 ml pyrogenfreier 0,9%iger Natriumchlorid-Lösung entfernt und die Säule abschließend mit 50 ml 20 mM Natriumphosphat-Puffer pH 7,4 äquilibriert. Die Waschgänge und die Äquilibrierung erfolgen ebenfalls mit einer Flußgeschwindigkeit von 5 ml/min. Ein Aliquot des letzten Eluates wird entnommen und auf Pyrogenfreiheit geprüft. Aus heparinisiertem (25 IU/ml Li-Heparin) Vollblut gesunder Spender wird durch Zentrifugation mit 1500 x g für 15 Minuten Plasma gewonnen. Dem Plasma wird sofort nach der Gewinnung Endotoxin (z.B. Lipopolysaccharid aus E. coli, 055:B5, Sigma) zugesetzt und die Endotoxinmenge bestimmt. Je 20 ml Plasma mit einem Gesamtendotoxingehalt von 10.000 Einheiten (EU) werden mit einer Flußgeschwindigkeit von 2 ml/min über die Säule gepumpt. Der Durchlauf wird aufgefangen. Nach Durchlauf des Plasmas wird die Säule mit 20 ml 20 mM pyrogenfreiem Natriumphosphat-Puffer nachgespült. Die Spüllösung wird ebenfalls aufgefangen.

Aus der Differenz der eingesetzten Endotoxinmenge vor der Säule und der Endotoxinmenge der vereinigten Eluate nach der Säule wird die Adsorptionskapazität der Albumin-Acrylharzbeads bestimmt. Kontrollexperimente werden mit einer Säule aus 5 ml Acrylharzbeads ohne Albuminbeschichtung unter gleichen Versuchsbedingungen durchgeführt.

Die Endotoxinbestimmung erfolgte in allen Experimenten mittels eines turbidimetrischen kinetischen Limulus Amöbocytenlysat Testes (LAL-Test siehe: Remillard, J.F. et al. 1987. In: Watson, S.W., Levin, J., Novitsky, T.J. (eds): "Detection of bacterial endotoxins with the Limulus Amebocyte Lysate Test". New York, Alan R. Riss, Inc. pp 197-210).

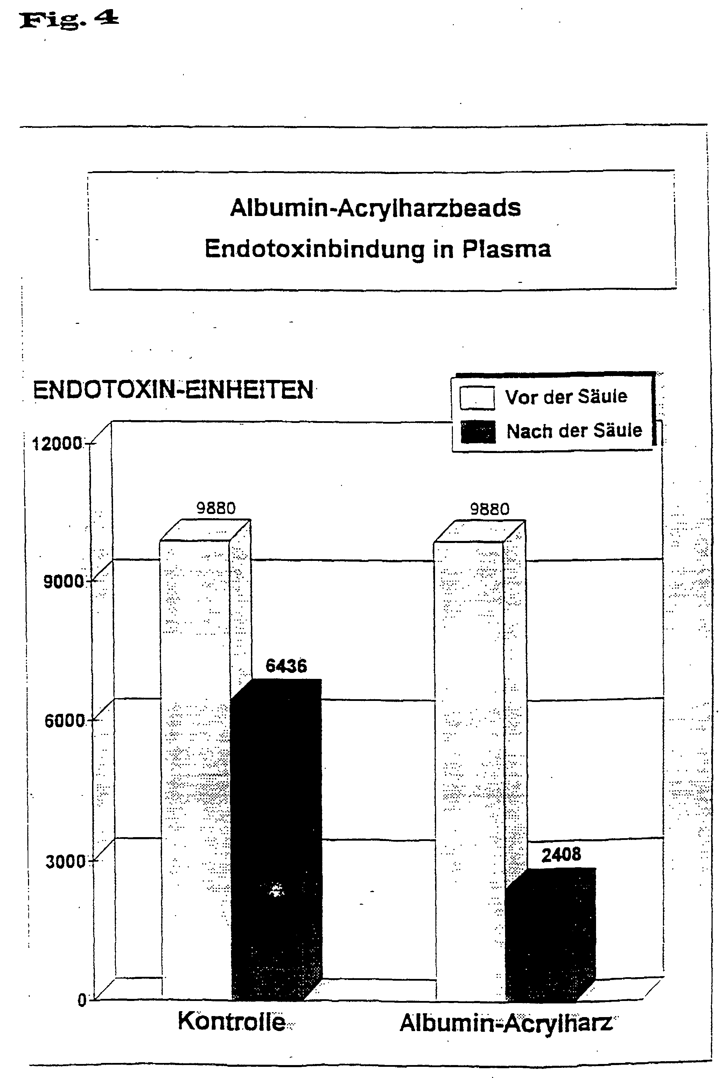

Tabelle 2 und Fig. 4 fassen die typischerweise in den Experimenten erzielten Ergebnisse zusammen. Gegenüber der Kontrolle zeigen die mit Albumin beschichteten Acrylharzbeads eine deutlich erhöhte Endotoxinbindung.

**Tabelle 2**

| Adsorptive Entfernung von Endotoxinen aus Humanplasma mit Albumin-Acrylharzbeads. | | | |
|---|---|---|---|
| **Gel n=5** | **Gesamtendotoxin vor der Säule** | **Gesamtendotoxin nach der Säule** | **Bindung (%)** |
| Albumin-Acrylharzbeads | 9.880 EU | 2.408 EU | 76 |
| Kontrollbeads | 9.880 EU | 6.436 EU | 35 |

Die kovalente Immobilisierung von Albumin auf den Oberflächen fester Trägermaterialien ist auch deshalb besonders vorteilhaft, da durch die Art der chemischen Kopplung bestimmte Bindungsregionen des Albuminmoleküls exponiert werden können. Enthält z. B. die Oberfläche des festen Trägers Aminogruppen, so wird, bei Verwendung von Carbodiimid als Kopplungsreagenz, das Albuminmolekül bevorzugt über Strukturbereiche mit Carboxylgruppen an den Träger gebunden. Diese Regionen stehen zur Adsorption nicht mehr zur Verfügung.

Es ist ersichtlich, daß das über Carboxylgruppen immobilisierte Albumin ausgeprägte Bindungseigenschaften hinsichtlich der untersuchten Substanzen besitzt, da es im Zuge der Immobilisierung kationischen Charakter erhält.

Die Bindung des Albumins erfolgt über die Amingruppen des Trägers. Ein weiterer Vorteil des erfindungsgemäßen Adsorbers ist sein Vollblutverträglichkeit. Ein Maß dafür ist die Abnahme von Thrombozyten, die auf der Säule hängen bleiben. Um die verbesserte Vollblutverträglichkeit zu zeigen, wird im folgenden die Thrombocytenadhäsion in Heparinplasma in Anwesenheit von LPS (500 pg/ml) anhand eines Vergleichsversuchs untersucht.

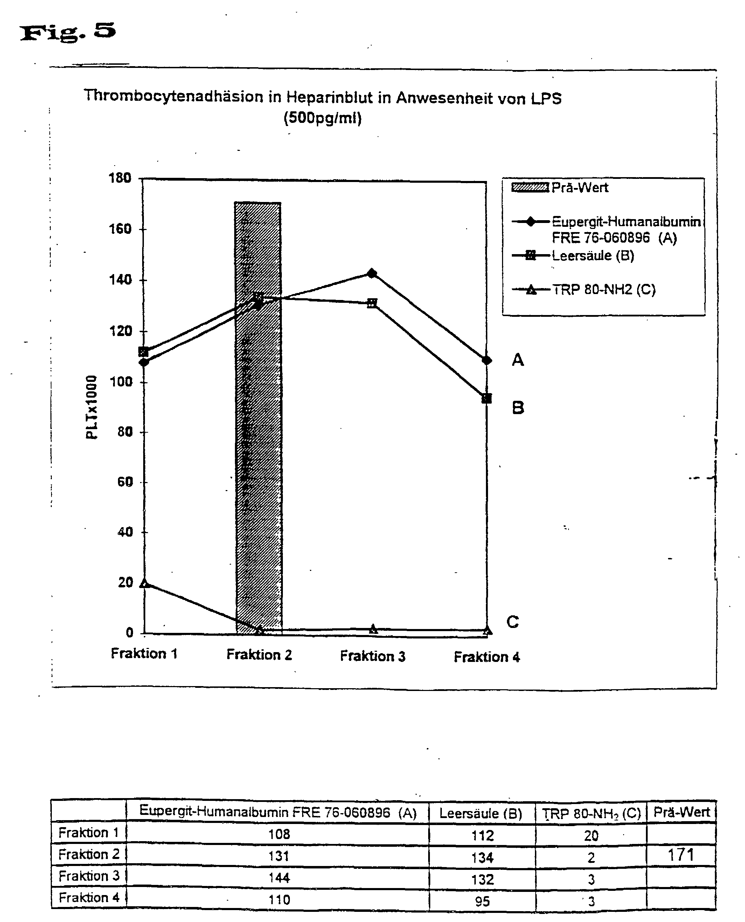

Fig. 5 zeigt beispielhaft die Eigenschaften des erfindungsgemäßen Albumin-Adsorbermateriales anhand des Thrombocytenverlustes des perfundierten Vollblutes. Die Hämokompatibilität des Adsorbers ist um so besser, je geringer der Abfall der Zellzahl in Bezug zum Prä-Wert ausfällt. Kurve A zeigt den Verlauf des Thrombocytenabfalles mit dem erfindungsgemäßen Albumin-Adsorbermaterial. Kurve B ist eine Kontrollkurve mit einer Säule ohne Adsorbermaterial. Kurve C zeigt den Verlauf des Thrombocytenabfalles mit einem Adsorbermaterial bekannt schlechter Hämokompatibilität.

| Versuchsbeschreibung: | |
|---|---|
| Säulen | A: Eupergit-Humanalbumin, Charge |
| | FRE 76-060896 |
| | B: Leersäule |
| | C: TRP 80-NH2 |
| | |
| Gelmenge | 4ml |
| | |
| Spüllösung | 500ml 0,9% NaCl-Lsg. |
| | 500ml 0,9% NaCI-Lsg. + 5 I.E./ml Na-Heparin |
| | |
| Spülfluß | 900 ml/h |
| | |
| Blutvolumen | 12 ml |
| | |
| Heparindosierung | 25 I.E./ml |
| | |
| verwendetes Heparin | Natrium-Heparin, 5000 I.E./0,2ml, |
| | B. Braun-Melsungen |
| | |
| Blutfluß | 30 ml/h |
| | |
| Fraktionen | 4 Fraktionen zu je 2 ml. |

### Versuchsdurchführung:

Vollblut gesunder Spender wurde aus einer gestauten Armvene abgenommen und in mit Na-Heparin vorbereitete (Heparindosierung: 25 I.E. / ml Blut), sterile Falcon PP-Röhrchen überführt. Die Heparinblutspenden wurden anschließend mit 500 pg/ml LPS aus E.coli 055:B5 versetzt und 1 Stunde bei 37°C auf einem Rollenmischer vorinkubiert. Die mit dem Adsorbermaterial gepackte Säulen wurden vor dem Versuch mit den oben genannten Spüllösungen mit Hilfe einer Volumed-Infusionspumpe (Fresenius) gespült. Aus der Blutprobe wurden vor Versuchsbeginn ca. 2 ml abgenommen (Prä-Wert). Anschließend wurden mit Hilfe der Volumed-Pumpe über jede Säule 12 ml Blut gepumpt (Fluß s.o.). Fraktionen von je 2 ml wurden in Eppendorfgefäßen aufgefangen. Die Zellzahlbestimmungen erfolgten in allen Proben mit Hilfe eines "Sysmex K1000".

Eine weitere für therapeutische Zwecke besonders geeignete Eigenschaft des erfindungsgemäßen Adsorbers ist die Heißdampfsterilisierbarkeit des verwendeten Materials. Diese Methode der Heißdampfsterilisation bei 121°C und 1 bar Überdruck wird nicht von jedem Material toleriert, insbesondere ist keine Toleranz bei Proteinen zu erwarten. Erfindungsgemäß ist es gelungen, durch Mehrpunktanbindung diese Eigenschaft der Dampfsterilisierbarkeit zu erhalten. Die Mehrpunktbindung wird erreicht, indem die molaren Verhältnisse der Ausgangsstoffe derart gewählt werden, daß die molaren Gruppen des Trägermaterials zu der Albuminkonzentration im Überschuß eingesetzt werden, vorteilhafterweise im Verhältnis 50 bis 500 zu 1.

Zwar ist auch eine andere Art der Sterilisation denkbar, beispielsweise Ethylenoxid oder γ-Bestrahlung, doch ist dann nicht ausgeschlossen, daß Fremdstoffe oder Abbaustoffe an das Adsorbermaterial gelangen, die später den Patienten gefährden könnten. Daher werden heute medizinische Produkte zunehmend nur noch heißdampfsterilisiert. Problematisch ist diese Methode, wenn Proteine verwendet werden, da diese naturgemäß bei Hitze denaturieren. Erst durch die erfindungsgemäße Mehrpunktanbindung kann eine Denaturierung verhindert und eine Heißdampfsterilisierbarkeit ermöglicht werden.

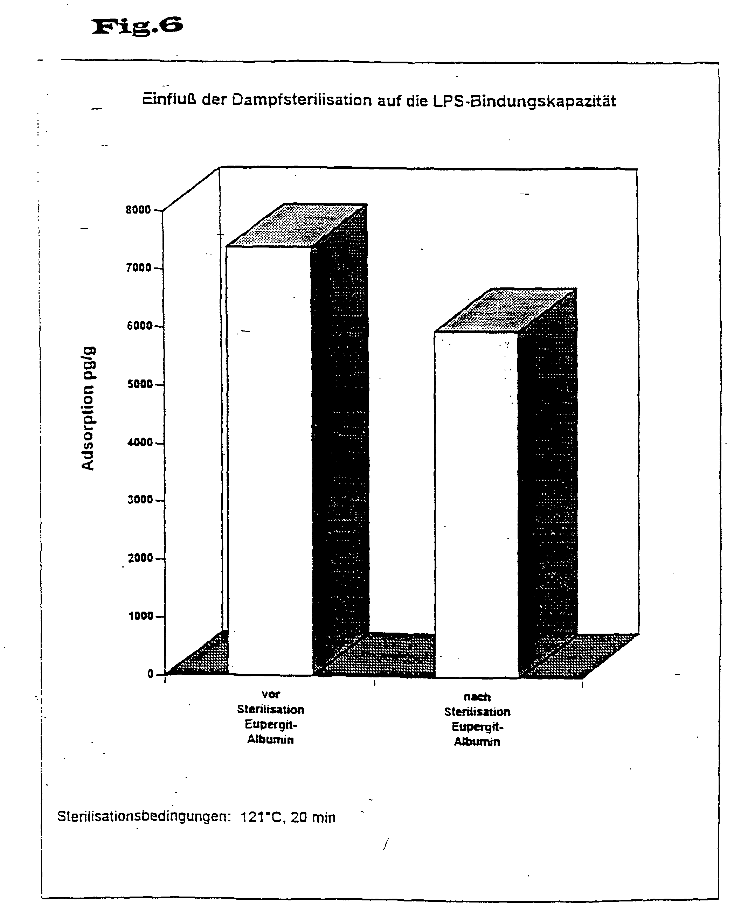

In Fig. 6 ist eine Graphik dargestellt, die die Lipopolysacharidbindung (Endotoxin) vor und nach der Sterilisation zeigt.

## Patentansprüche

1. Vorrichtung zur Reinigung proteinhaltiger Lösungen, wie Blut, Blutplasma oder Zellkulturmedien,
**gekennzeichnet durch**
ein biokompatibles Trägermaterial aus Kunststoffmaterial, wobei das Kunststoffmaterial aus der Gruppe Polyacrylate, Polymethacrylate, Polysulfon und Polyethersulfon, welche als funktionelle Gruppe Amine bzw. Amide trägt, ausgewählt ist, wobei diese mittels Peptidbindung mit Albumin kovalent beschichtet sind.

2. Vorrichtung nach Anspruch 1, dadurch gekennzeichnet, daß das Albumin über eine Mehrpunktbindung an das Kunststoffmaterial gebunden ist.

3. Vorrichtung nach Anspruch 1 oder 2, dadurch gekennzeichnet, daß das Trägermaterial in Form von Schüttungen von Beads, in Form von Membranen in Flach- oder Hohlfaserform oder in Form einer Folie eingesetzt werden.

4. Verfahren zur Herstellung eines Trägermaterials für eine Vorrichtung nach einem der Ansprüche 1-3, dadurch gekennzeichnet, daß die Mehrpunktbindung über eine Kopplerreagenz erzeugt wird, das lediglich die Carbonylfunktion des Albumins aktiviert und anschließend wieder abgespalten wird.

5. Verfahren nach Anspruch 4, dadurch gekennzeichnet, daß als Kopplerreagenz Carbodiimid oder N-Hydroxysuccinimid verwendet werden.

6. Verfahren nach einem der Ansprüche 4 oder 5, dadurch gekennzeichnet, daß die reaktiven Gruppen des Trägermaterials in einem molaren Verhältnis 50 bis 500 zu 1 zu der Albuminkonzentration verwendet werden.

7. Verfahren nach einem der vorangehenden Ansprüche, dadurch gekennzeichnet, daß das Kopplerreagenz bezogen auf die reaktiven Gruppen des Trägermaterials in gleicher bis 10-facher molarer Konzentration eingesetzt wird.

8. Verwendung einer Vorrichtung nach einem der vorangehenden Ansprüche zur Entfernung von Toxinen aus menschlichem Vollblut oder menschlichem Plasma.

9. Verwendung einer Vorrichtung nach einem der vorangehenden Ansprüche zur Entfernung von Toxinen aus Zellkulturmedien.

## Claims

1. Apparatus for the purification of proteic solutions such as blood, blood plasma or cell culture media,
**characterised by**
a biocompatible substrate of plastics material, said plastics material being selected from the polyacrylate, polymethacrylate, polysulphone and polyether sulphone group, which supports amines or amides as a functional group, these being covalently coated with albumin by means of peptide binding.

2. Apparatus according to Claim 1, characterised in that albumin is bound to the plastics material by multi-point binding.

3. Apparatus according to Claim 1 or 2, characterised in that the substrate or support material is used in the form of beds of beads, in the form of membranes of flat or hollow fibre form, or in the form of a sheet.

4. Process for the manufacture of a substrate or support material for an apparatus according to one of Claims 1 to 3, characterised in that the multi-point binding is generated by a coupling reagent that merely activates the carbonyl function of the albumin and is subsequently removed again.

5. Process according to Claim 4, characterised in that canbodiimide or N-hydroxysuccinimide is used as the coupling reagent.

6. Process according to one of Claims 4 or 5, characterised in that the reactive groups of the substrate or support material are used in a molar ratio of between 50 and 500 to 1 in relation to the albumin concentration.

7. Process according to one of the preceding Claims, characterised in that the coupling reagent is used in a molar concentration of the same value up to 10 times that value in relation to the reactive groups of the substrate or support material.

8. Application of an apparatus according to one of the preceding Claims for the removal of toxins from human whole blood or human plasma.

9. Application of an apparatus according to one of the preceding Claims for the removal of toxins from cell culture media.

## Revendications

1. Appareillage pour la purification de solutions protéiques, telles que du sang, du plasma sanguin ou des milieux de culture cellulaire,
**caractérisé par**
un support biocompatible en matériau plastique, le matériau plastique étant choisi parmi le groupe des polyacrylates, polyméthacrylates, polysulfone et polyéthersulfone, qui porte comme groupe fonctionnel des amines ou des amides, lesquels étant recouverts d'albumine de manière covalente au moyen d'une liaison péptidique.

2. Appareillage selon la revendication 1, caractérisé en ce que l'albumine est liée au matériau plastique moyennant une liaison à plusieurs points.

3. Appareillage selon les revendications 1 ou 2, caractérisé en ce que le matériau de support est utilisé sous forme de lits de perles, sous forme de membranes en forme plate ou à fibres creuses ou en :forme d'un film.

4. Procédé pour la préparation d'un matériau de support pour un appareillage selon l'une des revendications 1 à 3, caractérisé en ce que la liaison en plusieurs points est produite par un réactif de combinaison qui ne fait qu'activer la fonction de carbonyle de l'albumine et qui ensuite est de nouveau éliminé.

5. Procédé selon la revendication 4, caractérisé en ce que du carbodiimide ou du N-hydroxysuccinimide sont utilisés comme réactif de combinaison.

6. Procédé selon l'une quelconque des revendications 4 ou 5, caractérisé en ce que les groupes réactifs du matériau de support sont utilisés dans un rapport molaire entre 50 et 500 à 1 par rapport à la concentration d'albumine.

7. Procédé selon l'une quelconque des revendications précédentes, caractérisé en ce que le réactif de combinaison rapporté au groupe réactif du matériau de support est utilisé en concentration molaire égale à 10 fois la concentration.

8. Utilisation d'un appareillage selon l'une quelconque des revendications précédentes pour l'élimination de toxines d'un sang humain entier ou d'un plasma humain.

9. Utilisation d'un appareillage selon l'une quelconque des revendications précédentes pour l'élimination de toxines à partir de milieux de culture cellulaire.
